# EUROPÄISCHE PATENTSCHRIFT

(11) **EP 0 627 621 B1**
(45) Veröffentlichungstag und Bekanntmachung des Hinweises auf die Patenterteilung: **21.05.1997**
(21) Anmeldenummer: 93810866.9
(22) Anmeldetag: 09.12.1993
(51) Int. Cl.: G01N 27/416

(54) **Einrichtung und Verfahren zur Untersuchung des Stoffwechsels von Zellen**
Device and method for the examination of metabolism of cells
Méthode et appareil pour l'examen du métabolisme dans des cellules

(30) Priorität: 04.06.1993 EP 93810405
(43) Veröffentlichungstag der Anmeldung: 07.12.1994
(73) Patentinhaber: AVL Medical Instruments AG, 8207 Schaffhausen (CH)
(72) Erfinder: Hatschek, Rudolf A., Dr., CH-1700 Fribourg (CH); Heitz, Erich W. F., Dr. med., CH-8207 Schaffhausen (CH)
(74) Vertreter: Krause, Walter, Dr. Dipl.-Ing.

(56) Entgegenhaltungen:
- EP-A- 0 026 591
- EP-A- 0 028 793
- EP-A- 0 471 330

## Beschreibung

Die Erfindung betrifft eine Einrichtung zur Untersuchung des Stoffwechsels von an eine Flüssigkeit angrenzenden Zellen.

Bei den Zellen kann es sich um Mikroorganismen oder um andere lebende Zellen handeln, die einem menschlichen, tierischen oder pflanzlichen Organismus entnommen wurden und/oder von einem solchen abstammen. Die Flüssigkeit kann Wasser aufweisen und zum Beispiel aus einer wässerigen Lösung bestehen. Beim untersuchten Stoffwechsel der Zellen können durch mindestens einen Stoffwechselvorgang von den Zellen in der an diese angrenzenden Flüssigkeit Protonen freigesetzt und/oder eventuell durch mindestens einen Stoffwechselvorgang vorher in der Flüssigkeit vorhandene Protonen neutralisiert und/oder von den Zellen aufgenommen werden. Die Freisetzung von Protonen kann zum Beispiel durch direkte Abgabe von Protonen erfolgen. Ferner können die Zellen Stoffe an die Flüssigkeit abgeben, die erst in dieser Protonen freisetzen. Die Zellen produzieren zum Beispiel bei vielen Stoffwechselvorgängen Kohlendioxid, das dann in der die Zellen umgebenden Flüssigkeit Kohlensäure bildet. Zudem können in den Zellen niedermolekulare, aliphatische Hydroxisäuren - wie Milchsäure - entstehen, die dann durch die Zellmembran hindurch an die Flüssigkeit abgegeben werden. Im übrigen sind die in der Flüssigkeit ursprünglich vorhandenen und/oder während einer Untersuchung freigesetzten Protonen selbstverständlich wie üblich mindestens zum Teil an Wassermoleküle angelagert.

Aus den Publikationen "Light-Adressable Potentiometric Sensor for Biochemical Systems", Dean G. Hafeman, J. Wallace Parce, Harden M. McConnell, Science, Band 240, 1988, Seiten 1182 - 1185, und "Silicon Micromachining in the Fabrication of Biosensors Using Living Cells", Luc J. Bousse, J. Wallace Parce, John C. Owicki, and Karen M. Kercso, Tech. Dig. IEEE Solid State Sensor Workshop, 1990, Seiten 173 - 176, bekannte Einrichtungen zur Untersuchung des Stoffwechsels von lebenden Zellen besitzen eine Kammer zum Aufnehmen der Zellen und einer wasserhaltigen Flüssigkeit. Die Einrichtungen weisen Sensormittel zum Messen des p_{H}-Wertes der Flüssigkeit auf. Die Sensormittel besitzen einen Silicium-Halbleiter-Sensor, dessen beim Messen an die Flüssigkeit angrenzende Oberfläche durch eine elektrisch isolierende Schicht Siliciumoxinitrid oder Siliciumnitrid gebildet ist. Die Sensormittel besitzen noch mindestens eine Leuchtdiode, zum Beleuchten des Sensors und mindestens eine in die Flüssigkeit eintauchende Elektrode, um zwischen der Flüssigkeit und dem Sensor eine Potentialdifferenz zu erzeugen. Bei einer Untersuchung wird intermittierend eine Nährflüssigkeit durch die Kammer hindurchgeleitet und während den Strömungsunterbrüchen der vom Sensor erzeugte Fotostrom gemessen. Dieser gibt dann ein Mass für die durch den Stoffwechsel der Zellen verursachte Änderung des p_{H}-Wertes und damit auch für den Stoffwechsel selbst.

Viele Zellen sind empfindlich auf Änderungen des p_{H}-Wertes der sie umgebenden Flüssigkeit. Da der p_{H}-Wert bei den bekannten Einrichtungen während einer Messung ändert, haben die bekannten Einrichtungen und ihr Betrieb den Nachteil, dass die Flüssigkeit während eines grossen Teils der Messzeit einen für die Zellen ungünstigen p_{H}-Wert hat.

Die bei vielen Stoffwechselvorgängen in der die Zellen umgebenden Flüssigkeit entstehende Kohlensäure dissoziert teilweise in Protonen und Bicarbonat. Das letztere kann zusammen mit der undissozierten Kohlensäure als Puffer wirken, die Änderung des p_{H}-Wertes reduzieren und das Messergebnis verfälschen. Damit überhaupt einigermassen reproduzierbare Messungen möglich sind, wird der Flüssigkeit bei der Benutzung einer etwa gemäss den zitierten Publikationen ausgebildeten Einrichtung in der Praxis üblicherweise eine Puffersubstanz beigefügt. Dies bewirkt jedoch eine Vergrösserung der erforderlichen Messzeit, was vor allem bei der Untersuchung von schnell in den Zellen ablaufenden Vorgängen nachteilig ist.

Ein gewisser Nachteil der bekannten Einrichtungen besteht auch noch darin, dass die Sensormittel zum Messen des p_{H}-Wertes relativ viele verschiedene Teile, nämlich zusätzlich zum Silicium-Sensor noch mindestens eine Leuchtdiode und mindestens eine Elektrode benötigen.

Die Publikation "Preparation of Iridium Oxide and its Application in Sensor-Actuator Systems", W. Olthuis, J.C. van Kerkhof, P. Berveld, M. Bos, W.E. van der Linden, Sensors and Actuators B, 4, 1991, Seiten 151 - 156, Elsevier Sequoia, offenbart eine Einrichtung für die coulometrische Titration mit einem mit einer Iridiumoxidschicht versehenen Körper, der als Elektrode für die Abgabe und Aufnahme von Wasserstoffionen dient. Die Publikation enthält keinen Hinweis dafür, die beschriebene Einrichtung zum Untersuchen des Stoffwechsels von Zellen zu verwenden. Da der p_{H}-Wert während einer Titration ändert, hätte eine solche bei der Untersuchung des Stoffwechsels von Zellen zudem analog wie die aus den vorher zitierten Publikationen von Hafeman et al. und von Bousse et al. bekannte Messung der Änderung des p_{H}-Wertes den Nachteil, dass die die Zellen umgebende Flüssigkeit während der Messung nicht den optimalen p_{H}-Wert hat.

Die Herstellung von Iridiumoxidschichten durch Verfahren der in der zitierten Publikation von Olthuis et al. beschriebenen Art führt gemäß eigenen Studien und Untersuchung zu polykristallinen, dunklen, weitgehend opaken Oxidschichten. Solche Iridiumoxidschichten haben bei der Verwendung zum Protonenaustausch oder zur Messung des pH-Werts noch die Nachteile, daß ihre Protonenaustauschrate bzw. Meßempfindlichkeit im Verlauf der Zeit relativ stark ändert und daß sie auch nicht für Einrichtungen geeignet, bei denen die Oxidschicht beispielsweise für zusätzliche mikroskopische oder optische Untersuchungen lichtdurchlässig sein sollte.

Aus der EP-A 0 026 591 ist eine Vorrichtung zur automatischen Regelung des pH-Wertes eines elektrolythältigen Strömungsmediums bekannt, mit welchem eine Neutralisierung des Strömungsmediums angestrebt wird. Das Medium durchfließt dabei einen von einer flächigen Kathode und einer ionenselektiven Membran begrenzten ersten Teilraum einer Elektrolytzelle und wird durch eine ausgangsseitig angeordnete pH-Elektrode vermessen. Ein von der ionenselektiven Membran und einer flächigen Anode begrenzter zweiter Teilraum der Elektrolytzelle wird von einer wäßrigen NaCl-Lösung durchflossen. Der gemessene pH-Wert des Strömungsmediums wird mit einem vorgegebenen Sollwert verglichen. Zur Erreichung des gewünschten pH-Wertes bzw. der Neutralisierung des Mediums, wird der an der Anode und der Kathode der Zelle anliegende Strom auf der Basis des Vergleichswertes zwischen Soll- und Istwert des pH-Wertes justiert.

Der Erfindung liegt die Aufgabe zugrunde, eine Einrichtung für die Untersuchung des Stoffwechsels von Zellen zu schaffen, die Nachteile der bekannten Einrichtungen behebt und insbesondere ermöglicht, die Menge der von den Zellen bei mindestens einem Stoffwechselvorgang in der Flüssigkeit freigesetzten und/oder vielleicht die Menge der der Flüssigkeit durch Neutralisation oder Stoffaufnahme entzogenen Protonen zu messen, ohne daß sich der pH-Wert der Flüssigkeit während der Untersuchung in einer für die Zellen ungünstigen Weise ändert. Zudem soll ermöglicht werden, daß die Messungen nötigenfalls sehr schnell durchgeführt werden können.

Die gestellte Aufgabe wird gemäß der Erfindung durch eine Einrichtung mit den Merkmalen des Anspruchs 1 gelöst.

Die Erfindung betrifft des weiteren ein Verfahren gemäß dem Anspruch 10.

Vorteilhafte Ausgestaltungen der Einrichtung gehen aus den abhängigen Ansprüchen hervor.

Die Zellen können bei einer Untersuchung an einer Oberfläche der Einrichtung immobilisiert sein und dann mindestens mit ihren nicht an der Oberfläche der Einrichtung und nicht an einer anderen Zelle anliegenden Oberflächenbereichen an die Flüssigkeit angrenzen. Die Zellen können jedoch ebentuell auch teilweise oder ganz in der Flüssigkeit suspendiert sein. Die Flüssigkeit kann also die Zellen mindestens im wesentlichen umschliessen.

Bei der Benutzung der erfindungsgemässen Einrichtung können die p_{H}-Sensormittel, die zum Protonenaustausch, d.h. als Protonendonator und/oder -akzeptor ausgebildete Steuerelektrode und die Gegenelektrode in Kontakt mit der Flüssigkeit gebracht werden. Ferner kann mit den p_{H}-Sensormitteln der p_{H}-Wert der Flüssigkeit gemessen werden. Des weitern kann von den elektronischen Schaltungsmitteln ein von der Steuerelektrode zur Gegenelektrode und/oder in umgekehrter Richtung durch die Flüssigkeit fliessender Strom erzeugt werden, so dass die Steuerelektrode Protonen mit der Flüssigkeit austauscht, d.h. abhängig von der Stromrichtung Protonen abgibt oder aufnimmt. Die Schaltungsmittel können den Strom derart regeln, dass der p_{H}-Wert gleich einem vorgegebenen Sollwert ist. Die Schaltungsmittel können zudem den durch die Flüssigkeit fliessenden elektrischen Strom und/oder eine mit diesem Strom verknüpfte Messgrösse ermitteln.

Der Sollwert, auf den der p_{H}-Wert während einer Untersuchung oder Messung geregelt wird, kann mindestens während einer vorgesehenen Messzeit konstant und gleich dem Optimalwert für die untersuchten Zellen sein. Es sei angemerkt, dass der optimale p_{H}-Wert für verschiedene Arten von Zellen verschieden sein kann. Die Einrichtung kann daher mit manuell einstellbaren Stellmitteln versehen sein, mit denen ein dem optimalen p_{H}-Wert einer zu untersuchenden Art von Zellen entsprechender Sollwert manuell eingestellt werden kann.

Bei einer bevorzugten Ausgestaltung der Einrichtung können deren Schaltungsmittel nicht nur die Stärke des durch die Flüssigkeit fliessenden elektrischen Stromes automatisch regeln, sondern auch dessen Richtung automatisch derart einstellen, dass die Steuerelektrode je nach Bedarf Protonen abgeben oder aufnehmen kann. Eventuell können manuell betätigbare Stellmittel vorgesehen sein, die ermöglichen die Stromrichtung wahlweise manuell oder automatisch einzustellen.

Der zum Regeln des p_{H}-Wertes durch die Flüssigkeit geleitete Strom gibt ein Mass für die Menge und Anzahl der von den Zellen pro Zeiteinheit in der Flüssigkeit freigesetzten und/oder abgebauten Protonen und ist bei auf einen konstanten Sollwert geregelten p_{H}-Wert proportional zur Rate der freigesetzten und/oder abgebauten Protonen.

Die Einrichtung kann zum Beispiel ausgebildet sein, um mit mindestens einem manuell betätigbaren Betätigungsorgan und/oder mit automatisch arbeitenden Schaltungsmitteln einen Anfangszeitpunkt für eine Messung und die Dauer einer Mess- und/oder Integrationszeit festzulegen. Die Schaltungsmittel können des weiteren ausgebildet sein, um die Stärke des während der Mess- und/oder Integrationszeit durch die Flüssigkeit fliessenden elektrischen Stroms zu integrieren.

Falls der Istwert des p_{H}-Wertes beim Regeln um den Sollwert herum pendelt und falls deshalb die Stromrichtung beim Regeln geändert wird, kann der Stromstärke beim Integrieren abhängig von der Stromrichtung ein positives oder negatives Vorzeichen zugeordnet werden. Das Integral gibt dann ein Mass für die den Strom während der Mess- und/oder Integrationszeit bildende elektrische Ladungsmenge und für die von den Zellen freigesetzte und/oder abgebaute Menge von Protonen. Wenn der p_{H}-Wert während der Mess- und/oder Integrationszeit auf einen konstanten Sollwert geregelt wird, ist das Integral und die Ladungsmenge proportional zu der in der Flüssigkeit freigesetzten und/oder abgebauten Menge von Protonen.

Die zum Messen des p_{H}-Wertes dienenden p_{H}-Sensormittel der Einrichtung weisen vorzugsweise eine p_{H}-Messelektrode auf, die bei einer Untersuchung in Kontakt mit der an die Zellen angrenzende Flüssigkeit steht und deren elektrisches Potential gegenüber der Flüssigkeit vom p_{H}-Wert der letzteren abhängig ist. Die p_{H}-Sensormittel weisen ferner vorzugsweise eine Referenzelektrode auf, deren elektrisches Potential gegenüber der Flüssigkeit mindestens im wesentlichen unabhängig vom p_{H}-Wert ist und nämlich bei Änderungen des p_{H}-Wert entweder konstant bleibt oder höchstens wesentlich weniger ändert als das Potential der p_{H}-Messelektrode

Die Einrichtung weist vorzugsweise einen zum Tragen der genannten Elektroden ausgebildeten Träger mit einem elektrisch isolierenden Trägerteil auf. Der isolierende Trägerteil kann zum Beispiel durch ein einstückiges, im wesentlichen ebenes Plättchen gebildet sein, das aus einem kristallinen Material, wie Aluminiumoxid, nämlich einem Stück eines künstlichen, durchsichtigen, farblosen Saphirs besteht. Der isolierende Trägerteil besitzt beispielsweise eine ebene Oberfläche, auf welcher elektrisch leitende und/oder halbleitende, durch Zwischenräume voneinander getrennte Schichten aufgebracht sind, welche die genannten Elektroden oder mindestens deren freie, bei einer Untersuchung von Zellen in Kontakt mit der Flüssigkeit bringbaren Oberflächenbereiche bilden.

Die p_{H}-Messelektrode und die Steuerelektrode weisen vorzugsweise eine ihre freie Oberfläche bildende, bei einer Untersuchung an die Flüssigkeit angrenzende Schicht aus mindestens einem Metalloxid auf. Diese Metalloxidschicht soll mindestens einigermassen elektrisch leitend und/oder halbleitend sein.

Die Metalloxidschicht der p_{H}-Messelektrode und der Steuerelektrode besteht vorzugsweise aus mindestens einem Oxid von Iridium oder Palladium. Die Metalloxidschicht der Steuerelektrode kann jedoch aus mindestens einem Oxid von mindestens einem der Metalle Zirkon, Niob, Molybdän, Technetium, Ruthenium, Rhodium, Tantal, Wolfram, Rhenium, Osmium, Platin bestehen. Diese Metalle gehören wie Iridium und Palladium zur fünften oder sechsten Periode und - mit Ausnahme von Zirkon - zur Nebengruppe 5a oder 6a oder 7a oder 8 des Periodensystems der chemischen Elemente. Dabei besteht die Oxidschicht vorzugsweise aus einem Oxid oder aus Oxiden von nur einem einzigen der genannten Metalle.

Eine aus Iridiumoxid bestehende Schicht ist besonders gut als Protonendonator und/oder -akzeptor zur Bildung einer Steuerelektrode und auch gut zur Bildung einer p_{H}-Messelektrode geeignet, weil sie zum Protonenaustausch nur ein kleines elektrisches Elektrodenpotential - d.h. nur ein kleines zwischen der Elektrode und der Flüssigkeit vorhandenes, elektrisches Potential - erfordert. Dieses liegt beispielsweise unter dem Potential, bei welchem allenfalls in der Flüssigkeit vorhandene Chlorionen oxidiert und als Chlor freigesetzt werden. Ferner ist bei einer Elektrode mit einer Schicht aus Iridiumoxid das zum Protonenaustausch erforderliche Elektrodenpotential auch kleiner als das eine elektrolytische Zerlegung von Wasser bewirkende Potential. Auch eine aus Palladiumoxid bestehende Oxidschicht ist noch gut für die Bildung einer zum Protonenaustausch dienenden Steuerelektrode und einer p_{H}-Messelektrode geeignet.

Falls die Oxidschicht der Steuerelektrode und der p_{H}-Messelektrode ein Oxid eines Metalls aufweist, das in verschiedenen Oxidationsstufen oxidierbar ist, soll vorzugsweise zumindest der die freie Oberfläche der Oxidschicht bildende und bei einer Untersuchung an die Flüssigkeit angrenzende Teil der Oxidschicht aus dem Oxid der höchstmöglichen Oxidationsstufe bestehen. Dies schützt die Oxidschicht gegen eine weitere Oxidation und trägt damit zur chemischen Stabilität der Oxidschicht bei. Im Falle einer Iridiumoxidschicht besteht also vorzugsweise zumindest der deren freie Oberfläche bildende Teil aus dem Oxid der höchsten Oxidationsstufe, d.h. aus IrO₂.

Auf dem elektrisch isolierenden Trägerteil ist vorzugsweise zwischen diesem und der zur p_{H}-Messelektrode bzw. zur Steuerelektrode gehörenden, halbleitenden Metalloxidschicht eine Auflage oder Zwischenlage aufgebracht, die aus einer elektrisch leitenden Metallschicht besteht. Diese Metallschicht besteht zum Beispiel aus dem Metall oder möglicherweise den Metallen, dessen Oxid die Metalloxidschicht bildet bzw. deren Oxide die Metalloxidschicht bilden. Die Metalloxidschicht kann dann zwischen einem der Auflage abgewandten, schichtförmigen, aus einem Oxid der höchsten Oxidationsstufe bestehenden Abschnitt und der Metallschicht noch einen an diese angrenzenden Übergangsabschnitt haben, der aus einem Oxid niedriger Oxidationsstufe besteht.

Falls die Metalloxidschicht der p_{H}-Messelektrode und/oder der Steuerelektrode aus Iridiumoxid besteht, kann die vorzugsweise vorhandene Metallschicht aus Iridium bestehen. In diesem Fall kann die Oxidschicht zwischen einem bei ihrer Oberfläche vorhandenen, aus IrO₂ bestehenden Bereich und der aus Iridium bestehenden Metallschicht eventuell noch einen Bereich aufweisen, der aus Ir₂O₃ besteht.

Bei einer bevorzugten Ausführungsform der erfindungsgemässen Einrichtung ist die Metalloxidschicht der p_{H}-Messelektrode und/oder der Steuerelektrode oder mindestens der den freien, bei einer Untersuchung und Messung in Kontakt mit der Flüssigkeit gelangende Oberflächenbereich dieser Elektrode(n) bildende Teil der betreffenden Metalloxidschicht einkristallin. Eine solche einkristalline Metalloxidschicht hat eine stabile Struktur. Ferner besitzt sie im Gegensatz zu einer polykristallinen Oxidschicht keine inneren Grenzflächen. Dadurch lässt sich erreichen, dass die Oxidschicht kompakt und vollständig frei von Spalten sowie Mikrospalten ist und bei sachgemässer Verwendung auch frei von Spalten bleibt. Wenn die einkristalline Metalloxidschicht bei der Verwendung der Einrichtung in Kontakt mit der Flüssigkeit gelangt, kann diese daher nicht in die Metalloxidschicht eindringen. All diese Eigenschaften tragen dazu bei, dass die eine einkristalline Metalloxidschicht aufweisende Elektrode bei den noch näher beschriebenen Untersuchungen und Messungen sowohl kurzfristig als auch langfristig ein stabiles und gut reproduzierbares Verhalten zeigt.

Die zusammen mit p_{H}-Messelektrode zur p_{H}-Messung dienende Referenzelektrode kann zum Beispiel mindestens zum Teil aus Silberchlorid oder Kalomel bestehen und eine auf dem erwähnten Trägerteil angeordnete, bei einer Untersuchung in Kontakt mit der Flüssigkeit bringbare Schicht aus Silberchlorid bzw. Kalomel aufweisen.

Die zusammen mit der Steuerelektrode zur Erzeugung eines durch die Flüssigkeit fliessenden Stromes dienende Gegenelektrode kann zum Beispiel mindestens zum Teil aus einem metallischen Material bestehen und eine auf dem erwähnten Trägerteil angeordnete Metallschicht aufweisen.

Bei einer vorteilhaften Ausgestaltung der Einrichtung kann der aus einem Plättchen bestehende Trägerteil zusammen mit mindestens einem anderen Teil einen Raum zum Aufnehmen der Zellen und der Flüssigkeit begrenzen, an den die Elektroden angrenzen. Dieser Raum besteht vorzugsweise aus einem freien, d.h. leeren und kein festes Material enthaltenden, allseitig dicht gegen die Umgebung abgeschlossenen Hohlraum. Die Elektroden können nahe beieinander angeordnet sowie relativ klein bemessen werden und beispielsweise innerhalb eines Hüllkreises oder Hüllquadrats liegen, dessen Durchmesser bzw. Seitenlänge höchstens 10 mm oder sogar nur höchstens 5 mm beträgt. Die rechtwinklig zu der die Elektroden tragenden Oberfläche des Trägers gemessene Höhe des Hohlraums kann vorzugsweise höchstens 3 mm und beispielsweise ungefähr 1 mm betragen. Diese Ausbildung und Bemessung der Einrichtung ermöglicht, relativ kleine Mengen von Zellen und Flüssigkeiten zu untersuchen. Ferner kann die Menge der von den Zellen in der Flüssigkeit freigesetzten und/oder abgebauten Protonen in einer kurzen Messzeit ausreichend genau gemessen werden. Man kann daher die Protonenmenge beispielsweise in einer nur höchstens 30 Sekunden oder sogar nur höchstens 10 Sekunden betragenden Messzeit ermitteln.

Die einkristalline Metalloxidschicht kann zum Beispiel im Hochvakuum durch ein dünnschichttechnologisches Verfahren hergestellt und gleichzeitig auf den Träger aufgebracht werden. Die Oxidschicht wird dabei vorzugsweise auf eine von einer metallischen Auflage des Trägers gebildete Oberfläche aufgebracht. Für die Herstellung einer Iridiumoxidschicht kann man zum Beispiel auf einen elektrisch isolierenden, mindestens bis zu einer Temperatur von 800°C beständigen, wie schon erwähnt beispielsweise aus einem Saphirplättchen bestehenden Trägerteil in einem ersten Aufdampfvorgang durch Aufdampfen von Iridium eine aus reinem Iridium bestehende, metallische Auflage bilden. In einem unmittelbar anschliessenden, zweiten Aufdampfvorgang kann man auf diese metallische Auflage weiter Iridium aufdampfen und gleichzeitig Sauerstoff in den den Trägerteil enthaltenden Raum einleiten. Der Trägerteil kann mindestens während des zweiten Aufdampfvorgangs auf eine ungefähr 600°C bis 800°C betragende Temperatur erhitzt werden. Beim zweiten Aufdampfvorgang bildet sich auf der metallischen Auflage dann eine einkristalline Iridiumoxidschicht. In analoger Weise kann man auch eine einkristalline Metalloxidschicht von einem der andern genannten Metalle bilden.

Wenn ein elektrisch isolierender Trägerteil zusätzlich zu mindestens einer eine Metalloxidschicht aufweisenden Elektrode beispielsweise noch eine Referenzelektrode aus Silberchlorid und/oder eine Gegenelektrode aus Platin trägt, kann man noch Silber bzw. Platin auf den Trägerteil aufdampfen und das Silber nachträglich mit einer chlorhaltigen Flüssigkeit in Silberchlorid umwandeln. Desgleichen kann man noch elektrisch leitend mit den verschiedenen Elektroden verbundene Leiterbahnen auf den Trägerteil aufdampfen. Dies ermöglicht eine kostengünstige Herstellung eines mehrere Elektroden tragenden Trägerteils.

Es besteht jedoch auch die Möglichkeit, eine Metalloxidschicht auf eine nicht durch Aufdampfen, sondern in irgend einer anderen Weise hergestellte, metallische Auflage eines Trägers aufzubringen. Ferner kann die Oxidschicht eventuell auch durch Züchten eines Oxid-Einkristalls und anschliessendes Zerschneiden und/oder Schleifen hergestellt werden. Die dabei gebildete Scheibe kann dann auf dem Träger befestigt werden.

Bei einer vorteilhaften Ausgestaltung der erfindungsgemässen Einrichtung beträgt die Dicke der Metalloxidschicht mindestens 50 nm und höchstens 1 mm. Wenn die Oxidschicht dünnschnittechnologisch hergestellt wird, kann ihre Dicke zum Beispiel bis 1000 nm betragen. Bei einer zu einer p_{H}-Messelektrode gehörenden Metalloxidschicht ist es günstig, diese nur etwa 100 nm bis 300 nm dick auszubilden. Eine solche, relativ geringe Dicke trägt zur Ermöglichung schneller Messungen bei. Bei einer zur Steuerelektrode gehörenden Metalloxidschicht ist es vorteilhaft, diese dicker auszubilden, damit sie genügende Mengen von Protonen abgeben und/oder aufnehmen kann. Die Oxidschicht kann dann zum Beispiel eine Dicke von mindestens 300 nm bis vielleicht etwa 600 nm oder mehr haben. Wenn eine Metalloxidschicht lichtdurchlässig sein soll, ist es vorteilhaft, die Oxidschicht höchstens etwa 300 nm dick zu machen, so dass sie möglichst wenig Licht absorbiert.

Eine zur Steuerelektrode und/oder zur p_{H}-Messelektrode gehörende Metalloxidschicht kann eventuell statt einkristallin auch polykristallin sein.

Ferner kann die p_{H}-Messelektrode und/oder die Steuerelektrode anstelle einer Metalloxidschicht eventuell eine mindestens zum Teil aus Platin bestehende Schicht aufweisen.

Zudem kann man die p_{H}-Sensormittel statt mit einer p_{H}-Messelektrode eventuell mit einem p_{H}-Silicium-Halbleiter-Sensor oder -Detektor ausrüsten, der eine dotierte Siliciumschicht und eine Siliciumnitrid- oder Siliciumoxinitridschicht besitzt. Der Sensor kann dann mehr oder weniger ähnlich ausgebildet sein, wie die Sensoren, die aus den weiter vorne zitierten Publikationen von Hafeman et al. und Bousse et al. bekannt sind. Die Referenzelektrode kann dann durch mindestens eine Elektrode ersetzt werden, welche die Funktion der aus den beiden letztgenannten Publikationen bekannten Elektroden ausüben kann.

Man kann vor dem Messen der von den Zellen freigesetzten Protonenmenge eine elektrolytische, aus einer wässerigen Lösung bestehende Flüssigkeit bereitstellen. Die Flüssigkeit soll - bevor sie in Kontakt mit den Zellen gelangt - vorzugsweise einen für die zu untersuchenden Zellen optimalen p_{H}-Wert und nur eine kleine oder gar keine Pufferkapazität aufweisen. Eine höchstens kleine oder fehlende Pufferkapazität trägt dazu bei, dass die Menge der während einer Messung von den Zellen freigesetzten Protonen schnell und genau gemessen werden kann.

Die bei einer Untersuchung an die Zellen angrenzende und diese im wesentlichen umgebende, wasserhaltige Flüssigkeit kann beim Beginn einer Untersuchung beispielsweise mindestens einen Nährstoff, wie Glukose und/ oder Glutamin enthalten. Ferner kann sie mindestens einen für das Wachstum der Zellen essentiellen Zusatzstoff und normalerweise mehrere solche - zum Beispiel Vitamine, Aminosäuren, Salze, Nukleoside, Hormone, exogene Wachstumsfaktoren - enthalten. Des weiteren kann die Flüssigkeit mindestens ein gelöstes Gas, wie Sauerstoff und/oder Kohlendioxid, enthalten.

Ferner kann der Flüssigkeit mindestens ein Teststofr beigefügt werden, dessen Wirkung auf die Zellen untersucht werden soll. Als Teststoffe können beispielsweise pharmazeutische Wirkstoffe oder in der Umwelt vorkommende Schadstoffe beigefügt werden.

Bei einer Untersuchung kann man dann zum Beispiel elektroanalytisch, nämlich coulometrisch bei konstant gehaltenem p_{H}-Wert die Menge der von Mikroorganismen oder sonstigen Zellen bei mindestens einem Stoffwechselvorgang an die Flüssigkeit abgegebenen Säure oder Säuren messen. Diese Säuremenge kann dann beispielsweise ein Mass für die Vitalität der Zellen geben.

Der Erfindungsgegenstand wird nun anhand der in der Zeichnung beschriebener Ausführungsbeispiele erläutert. In der Zeichnung zeigt
die Fig. 1 einen Schnitt durch einen Sensor mit einem mit einer Gruppe von Elektroden versehenen Träger und einem zur Aufnahme einer Flüssigkeit dienenden Hohlraum,
die Fig. 2 eine Draufsicht auf die mit Elektroden versehene Fläche des Trägers des in der Fig. 1 gezeichneten Sensors und ein Blockschema einer elektronischen Messvorrichtung,
die Fig. 3 einen in der Fig. 1 mit III bezeichneten Ausschnitt aus dem Sensor in grösserem Massstab und mit zusätzlichen Teilen und
die Fig. 4 eine Draufsicht auf einen mehrere Gruppen von Elektroden tragenden Träger.

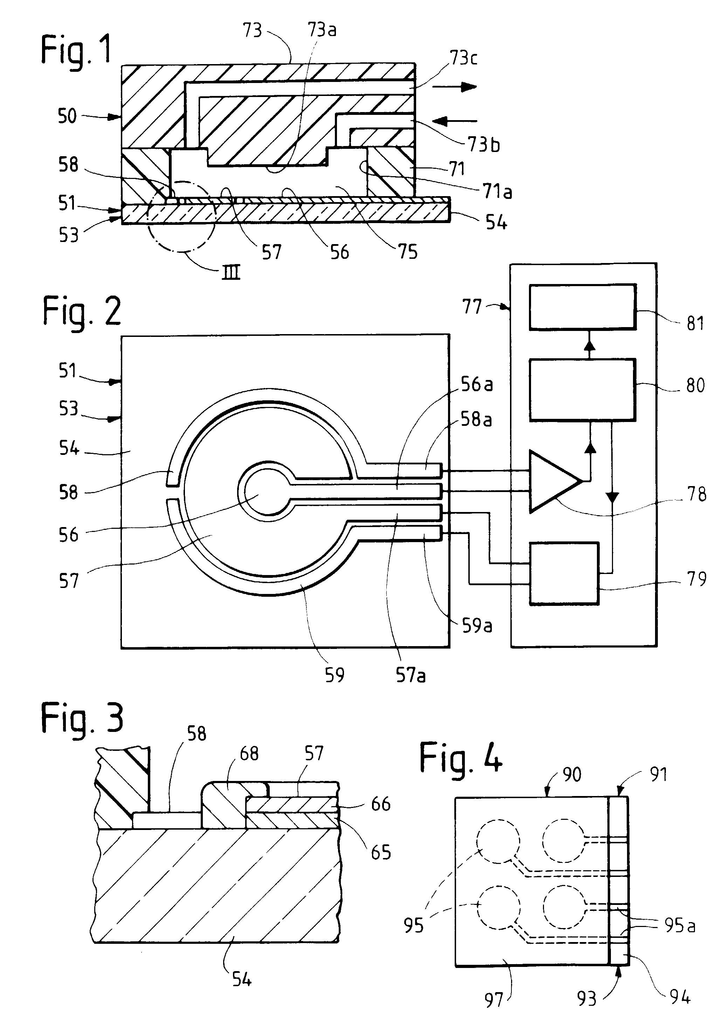

Zu den Figuren 1 bis 3 ist noch anzumerken, dass diese schematisiert und nicht massstäblich gezeichnet sind.

Eine Messeinrichtung für elektroanalytische, coulometrische Messungen zur Untersuchung des Stoffwechsels von Zellen besitzt eine in der Fig. 1 ersichtliche behälter- und/oder kammerförmige Vorrichtung, die einen Sensor 50 bildet. Dieser weist einen Körper 51 mit einem Träger 53 auf. Der Träger besitzt einen Trägerteil, der durch ein ebenes, elektrisch isolierendes, aus einer Saphirscheibe bestehendes, viereckiges Plättchen 54 gebildet ist. Das Plättchen 54 ist auf seiner sich in der Fig. 1 oben befindenden, ebenen Fläche mit vier in der Fig. 2 ersichtlichen Elektroden, nämlich einer p_{H}-Messelektrode 56, einer Steuer- und/oder Protonenaustauschelektrode elektrode 57, einer Referenzelektrode 58 und einer 59 versehen. Jede Elektrode ist elektrisch leitend mit einer auf dem Plättchen angeordneten Leiterbahn 56a bzw. 57a bzw. 58a bzw. 59a verbunden. Die Messelektrode 56 besitzt in der in der Fig. 2 gezeichneten Draufsicht einen kreisförmigen, aus einem vollen Kreis bestehenden Abschnitt. Die Steuer- und/oder Protonenaustauschelektrode 57 umschliesst die Messelektrode 56 annähernd, nämlich bis auf einen den Durchtritt der Leiterbahn 56a ermöglichenden Spalt. Die Steuer- und/oder Protonenaustauschelektrode 57 ist also etwa C-förmig, bildet einen durch den genannten Spalt unterbrochenen Kreisring und hat eine wesentlich grössere Oberfläche als die Messelektrode 56. Die Oberfläche der Steuer- und/oder Protonenaustauschelektrode 57 ist vorzugsweise mindestens fünfmal und beispielsweise siebenmal bis fünfzehnmal grösser als diejenige der p_{H}-Messelektrode 56. Jede der beiden Elektroden 58, 59 bildet einen im Vergleich zur Elektrode 57 schmalen, annähernd halbkreisförmigen Bogen und verläuft entlang von einem Abschnitt des Aussenrandes der Elektrode 57. Die vier Leiterbahnen verlaufen parallel zu einem eine Viereckseite des Plättchens 54 bildenden Abschnitt von dessen Rand.

Die Messelektrode 56 und die zum Teil auch in der Fig. 3 ersichtliche Steuer- und/oder Protonenaustauschelektrode 57 weisen je eine unmittelbar auf dem Plättchen 54 angeordnete, metallische, aus einer aufgedampften Schicht von reinem Iridium bestehende Auflage 65 und eine auf dieser aufliegende, einkristalline, aus Iridiumoxid bestehende Oxidschicht 66 auf. Die Leiterbahnen 56a und 57a bestehen aus Iridiumschichten, die mit der Auflage der Elektrode 56 bzw. 57 zusammenhängen. Die Referenzelektrode 58 besteht aus Silberchlorid und die mit ihr verbundene Leiterbahn 58a besteht aus Silber. Die Gegenelektrode 59 und die mit ihr verbundene Leiterbahn 59a bestehen aus Platin.

Zwischen den verschiedenen Elektroden und Leiterbahnen sind diese voneinander trennende, schmale Zwischenräume, d.h. Spalte vorhanden. Die Ränder der die Elektroden 56, 57 bildenden Auflagen 65 und Oxidschichten 66 sind zudem durch Abdeckungen - von denen eine in der Fig. 3 beim Aussenrand der Elektrode 57 ersichtlich und mit 68 bezeichnet ist - elektrisch isolierend und flüssigkeitsdicht abgedeckt und bei einer Untersuchung gegen die Fküssigkeit isoliert. Die Abdeckungen 68 bestehen beispielsweise aus aufgedampftem, reinem, undotiertem Silicium oder aus Siliciumdioxid.

Auf der mit den Elektroden versehenen Seite des Trägers 53 ist ein Plättchen 71 angeordnet, das ein durchgehendes, zur Gruppe der Elektroden 56, 57, 58, 59 konzentrisches Loch 71a besitzt, dessen Durchmesser ungefähr gleich dem Hüllkreis der Elektroden-Gruppe ist.

Ferner ist ein ebenfalls aus einem Plättchen bestehender Deckteil 73 vorhanden, der auf der dem Plättchen 54 abgewandten Seite des Plättchens 71 aufliegt und einen in dessen Loch 71a hineinragenden Vorsprung 73a hat. Die Plättchen 54, 71 und der Deckteil 73 begrenzen zusammen einen vom freien Teil des Lochs 71a gebildeten, dicht gegen die Umgebung abgeschlossenen Hohlraum 75 zur Aufnahme von Zellen und einer Flüssigkeit. Der Deckteil 73 besitzt zwei Durchgänge, die zwischen dem Rand des Vorsprungs 73a sowie dem Rand des Lochs 71a in den Hohlraum 75 münden und einen Einlass 73b sowie einen Auslass 73c für die zu Flüssigkeit bilden.

Das Plättchen 71 ist elektrisch isolierend und besteht zum Beispiel aus einem Kunststoff, könnte jedoch auch aus mineralischem Glas gebildet sein. Der Deckteil 73 besteht beispielsweise aus einem Kunststoff. Das Plättchen 54 und der Deckteil 73 sind durch Befestigungsmittel - zum Beispiel durch eine Klemmvorrichtung - lösbar zusammengehalten. Das Plättchen 71 kann unlösbar mit dem Plättchen 54 oder mit dem Deckteil 73 verbunden oder sowohl vom Plättchen 54 als auch vom Deckteil 71 lösbar zwischen diesem und dem Plättchen 54 eingespannt sein. Im übrigen kann das Plättchen 71 eventuell gemäss den Figuren 1 und 3 auf dem äusseren Randbereich der Elektroden 58, 59 aufliegen, soll diese aber nicht vollständig abdecken, so dass alle Elektroden 56, 57, 58, 59 an den Hohlraum 75 angrenzen. Der sich in der Draufsicht innerhalb des Hohlraums 75 befindende Abschnitt der aus Iridium bestehenden Leiterbahn 56a ist durch eine beispielsweise aus aufgedampftem Silicium bestehende Isolation gegen den Hohlraum 75 abgedeckt. Es wäre jedoch auch möglich, dass die Elektrode 56 zusätzlich zu dem sich im Zentrum der andern Elektroden befindenden kreisförmigen Abschnitt auch noch einen mit diesem sammenhängenden, geraden, sich mindestens bis zum Rand des Hohlraums 75 erstreckenden, mit einer Iridiumoxidbeschichtung versehenen, nicht von einer Isolation bedeckten Abschnitt aufweist. Es sei hier angemerkt, dass die aufgedampften Elektroden in den Figuren 1 und 3 mit stark übertriebenen Dicken gezeichnet sind. Damit der Hohlraum 75 bei zusammengebautem Sensor dicht gegen die Umgebung abgeschlossen ist, kann eventuell noch eine Schicht von elektrisch isolierendem und elastisch deformierbarem Dichtungs- und Isoliermaterial auf dem die Elektroden 58, 59 umschliessenden, vom Plättchen 71 bedeckten Bereich des Plättchens 54 und zwischen den Leiterbahnen sowie auf diesen aufgebracht sein.

Die den Sensor 50 aufweisende Messeinrichtung besitzt eine elektronische Messvorrichtung 77, d.h. ein Messgerät, von dem in der Fig. 2 das Blockschema gezeichnet ist. Die Leiterbahnen 56a, 57a, 58a, 59a sind beispielsweise über eine Steckverbindung elektrisch leitend mit der elektronische Schaltungsmittel aufweisenden Messvorrichtung 77 verbunden. Diese enthält einen Messverstärker 78, dessen Eingänge mit der p_{H}-Messelektrode 56 und mit der Referenzelektrode 58 verbunden sind. Die Messvorrichtung 77 besitzt ferner eine elektrisch regelbare Stromquelle 79, deren Ausgänge mit der Steuer- und/oder Protonenaustauschelektrode 57 und mit der Gegenelektrode 59 verbunden sind. Zur Messvorrichtung 77 gehört ferner eine Mess- und Regelschaltung 80, die beispielsweise einen digital arbeitenden Prozessor besitzt. Des weiteren ist eine Anzeige- und/oder Registriervorrichtung 81 vorhanden. Die Mess- und Regelschaltung 80 ist mit einem Ausgang des Messverstärkers 78, mit einem Regelanschluss der Stromquelle 79 und mit der Anzeige- und/oder Registriervorrichtung 81 verbunden.

Ferner sind noch Aufbereitungs- und Zufuhrmittel vorhanden, um eine Flüssigkeit aufzubereiten, zu konditionieren und dem Hohlraum 75 zuzuführen. Die Aufbereitungsmittel können beispielsweise ausgebildet sein, um in der Flüssigkeit einen günstigen p_{H}-Wert einzustellen, um die Flüssigkeit auf eine gewünschte Temperatur zu erwärmen und/oder zu kühlen und um in der Flüssigkeit bestimmte Gas-Partialdrücke - insbesondere Sauerstoff- und/oder Kohlendioxid-Patialdrücke zu erzeugen. Ferner sind vorzugsweise eine Heiz- und/oder Kühlvorrichtung sowie eine Temperaturregelvorrichtung vorhanden, um den Sensor so und vor allem die Flüssigkeit sowie die Zellen, die sich bei einer Untersuchung im Hohlraum 75 befinden, auf einer gewünschten Temperatur zu halten. Des weiteren können noch Dielektrophorese-Mittel vorgesehen werden, um die Bewegung der Zellen im Hohlraum 75 derart zu beeinflussen, dass die Zellen bei vorgegebenen Bereichen der an den Hohlraum 75 angrenzenden Flächen angelagert und immobilisiert werden. Die Dielektrophorese-Mittel können beispielsweise Dielektrophorese-Elektroden aufweisen, die eine Vielzahl von Vorsprüngen mit Kanten, Ecken und/oder kleinen Krümmungsradien besitzen und derart angeordnet sowie mit einem Wechselspannungsgenerator verbunden sind, dass sie ein inhomogenes, elektrisches Wechselfeld im Hohlraum 75 erzeugen können. Die Dielektrophorese-Elektroden können durch Metallschichten gebildet sein, die beispielsweise an einer an den Hohlraum 75 angrenzenden Fläche des Deckteils 73 angeordnet sind. Eventuell können die Dielektrophorese-Elektroden stattdessen an der dem Hohlraum 75 abgewandten Seite des Plättchens 54 angeordnet werden, so dass sie durch dieses und durch die Elektroden 56, 57, 58, 59 und/oder durch die zwischen diesen vorhandenen Spalte hindurch ein zur Dielektrophorese dienendes, elektrisches Wechselfeld im Hohlraum 75 erzeugen.

Wenn die Elektroden 56, 57, 58, 59 in Kontakt mit einer wässerigen, elektrolytischen, Flüssigkeit gebracht werden, kann man mit der p_{H}-Messelektrode 56 und der Referenzelektrode 58 elektroanalytisch, beispielsweise potentiometrisch den p_{H}-Wert ermitteln. Das sich zwischen den beiden Elektroden 56 und 58 ergebende elektrische Potential ist mindestens in einem sich ungefähr von 4 bis 9 erstreckenden Bereich der p_{H}-Werte praktisch linear mit dem p_{H}-Wert verknüpft, wobei der p_{H}-Wert mit abnehmendem Potential grösser wird.

Wenn zwischen der Steuer- und/oder Protonenaustauschelektrode 57 und der Gegenelektrode 59 ein elektrischen Strom durch die wässerige, elektrolytische, Protonen enthaltende Flüssigkeit hindurch geleitet wird, kann das Iridiumoxid der Elektrode 57, abhängig von der Stromrichtung durch Redoxreaktionen Protonen aufnehmen oder abgeben. Diese Reaktionen können vereinfacht beschrieben werden durch die Formel

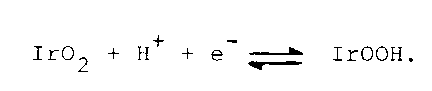

Je nachdem, ob die Elektrode 57 bei einer Messung vor allem Protonen abgeben oder vor allem Protonen aufnehmen soll, kann man das Iridium der Oxidschicht 66 nötigenfalls vor dieser Messung und nach einer allfälligen, vorangehenden Messung mit einem elektrischen, kathodischen Strom reduzieren bzw. mit einem elektrischen, anodischen Strom oxidieren und die Oxidschicht dabei mit Protonen sättigen bzw. von Protonen befreien. Es sei hierzu auch auf die schon zitierte Publikation von Olthuis et al. verwiesen.

Die den Sensor 50 und die elektronische Messvorrichtung 77 aufweisende Messeinrichtung kann beispielsweise verwendet werden, um eine von lebenden Zellen einer Zellkultur bei mindestens einem Stoffwechselvorgang ausgeschiedene Säuremenge zu messen. Für die Durchführung einer Messung kann man eine zu untersuchende Probe einer Suspension, die zum Beispiel eine konditionierte Nährflüssigkeit und in dieser suspendierte Zellen aufweist, durch den Einlass 73b in den Hohlraum 75 einbringen. Die Flüssigkeit kann zum Beispiel zusätzlich zu Wasser und mindestens einem in diesem gelösten Nährstoff abhängig von der vorgesehen Untersuchung noch gelösten Sauerstoff enthalten, jedoch beispielsweise beim Einbringen in den Hohlraum 75 frei von Kohlendioxid und Kohlensäure sein. Die eingebrachte Suspension soll den Hohlraum 75 beispielsweise vollständig füllen. Nach dem Einbringen der Suspension kann man im Sensor coulometrisch die Menge der während einer gewissen Messzeit von den Zellen freigesetzten und/oder abgebauten Protonen messen.

Die coulometrische Messung kann beispielsweise sofort nach dem Einbringen der Flüssigkeit und der Zellen beginnen. Man kann jedoch zwischen dem Einbringen der Zellen in den Hohlraum 75 und dem Messbeginn noch eine Warte- und Anlagerungszeit einschalten, während der die Zellen sich an einer an den Hohlraum 75 angrenzenden Fläche von mindestens einem der Teile des Sensors anlagern können, so dass die Zellen immobilisiert werden. Falls Dielektrophorese-Mittel vorhanden sind, kann die Anlagerung durch Dielektrophorese gesteuert und beschleunigt werden. Nötigenfalls kann man den Hohlraum 75 nach der Warte- und Anlagerungszeit vor dem Beginn der Messung noch mit frischer, konditionierter Flüssigkeit spülen, wobei die zum Spülen dienende Flüssigkeit durch den Auslass 73 aus dem Hohlraum 75 ausfliesst. Durch das Spülen kann erreicht werden, dass die Flüssigkeit beim anschliessenden Messbeginn eine genau definierte Zusammensetzung und beispielsweise auch einen genau definierten Gehalt von gelöstem Gas hat.

Die Temperatur der Flüssigkeit und der Zellen kann vor und während einer Messung auf den gewünschten Wert geregelt werden. Nötigenfalls kann man noch den Einlass 73b sowie den Auslass 73c oder mit dem Einlass sowie dem Auslass verbundene Leitungen abschliessen, so dass der Hohlraum 75 vollständig gegen die Umgebungsatmosphäre abeschlossen ist.

Bei der coulometrischen Messung misst die Mess- und Regelschaltung 80 die ein Mass für den p_{H}-Wert der Nährflüssigkeit gebende Potentialdifferenz zwischen der p_{H}-Messelektrode 56 und der Referenzelektrode 58. Ferner erzeugt die Stromquelle 79 einen zwischen der Steuer- und/oder Protonenaustauschelektrode 57 und der Gegenelektrode 59 durch die Nährflüssigkeit fliessenden, elektrischen Gleichstrom. Dieser kann gleichförmig sein oder aus einer Folge von Impulsen bestehen und ist derart gerichtet, dass die Steuer- und/oder Protonenaustausch elektrode 57 Protonen aus der Nährflüssigkeit aufnehmen kann. Die Mess- und Regelschaltung 80 regelt die Stromquelle 79 automatisch derart, dass die Protonenaufnahme der Elektrode 57 die Menge der von den Zellen freigesetzten Protonen kompensiert und dass der momentane P_{H}-Istwert der Nährflüssigkeit gleich einem vorgegebenen, mit manuell betätigbaren Einstellelementen eingestellten, für die Entwicklung der Zellen günstigen p_{H}-Sollwert ist. Eventuell kann die Mess- und Regelschaltung 80 die Stromrichtung im Bedarfsfall auch vorübergehend umkehren, so dass die Elektrode 57 vorübergehend Protonen abgibt. Die Regelung kann zum Beispiel unter Verwendung der sogenannten "Fuzzy" Logik erfolgen. Ferner misst und integriert die Mess- und Regelschaltung 80 den während einer vorgegebenen, beispielsweise manuell einstellbare Messzeit zwischen der Steuer- und/oder Protonenaustauschelektrode 57 und der Gegenelektrode durch die Nährflüssigkeit fliessenden Strom und ermittelt die während der Messzeit zur Konstanthaltung des p_{H}-Wertes der Nährflüssigkeit insgesamt durch diese hindurchgeleitete elektrischen Ladungsmenge. Diese gibt dann ein Mass für die von den Zellen direkt an die Flüssigkeit abgegebene und/oder in dieser gebildete Säuremenge. Die Anzeige- und/oder Registriervorrichtung 81 kann dann beispielsweise die Ladungsmenge oder eine zu dieser proportionale Grösse und eventuell noch den p_{H}-Wert anzeigen und/oder registrieren.

Nach der Durchführung einer Messung kann man eventuell den Honlraum 75 derart mit Flüssigkeit spülen, dass die Zellen noch im Hohlraum verbleiben, wobei eventuell die Zusammensetzung der Flüssigkeit oder die Temperatur oder ein anderer Verfahrensparameter geändert werden kann. Danach kann zum Beispiel eine andere coulometrische Messung durchgeführt werden.

Wenn die Untersuchung der im Hohlraum 75 vorhandenen Zellen abgeschlossen ist, kann man den Hohlraum 75 derart spülen, dass die Zellen ebenfalls herausgespült werden. Zudem kann man nötigenfalls den Deckteil 73 zum Reinigen der den Hohlraum 75 begrenzenden Teile vorübergehend vom Plättchen 54 trennen, die Steuer- und/oder Protonenaustauschelektrode 57 nötigenfalls elektrochemisch regenerieren,und danach wieder eine neue Probe in den Hohlraum 75 einbringen.

Man kann der Nährflüssigkeit, bevor sie für eine Messung in den Hohlraum 75 eingebracht wird, noch eine beispielsweise aus einem Arzneimittel oder einem Umweltgift bestehende Substanz beifügen, deren Wirkung auf die Zellen untersucht werden soll. Zudem kann eventuell für eine Messung zusätzlich zu Sauerstoff oder anstelle von solchem ein anderes Gas in der Nährflüssigkeit gelöst werden. Der Sensor 50 kann also gewissermassen einen kleinen Bioreaktor bilden, in dem man Zellen kultivieren und dabei deren Stoffwechsel untersuchen kann.

Der in der Fig. 4 ersichtliche Mehrfach-Sensor 90 besitzt einen Körper 91 mit einem Träger 93. Dieser besitzt als Hauptbestandteil ein aus einer Saphirscheibe bestehendes Plättchen 94, auf dem mehrere, nämlich zum Beispiel vier Elektroden-Gruppen 95 und Leiterbahnen-Gruppen 95a aufgebracht sind. Jede Elektroden- Gruppe 95 besitzt vier analog wie die Elektroden 56, 57, 58, 59 angeordnete Elektroden. Jede Leiterbahnen-Gruppe 95a weist vier Leiterbahnen auf, die je mit einer Elektrode verbunden sind. Gemäss der Fig. 4 können beispielsweise alle Leiterbahnen bei ein und derselben Viereckseite des viereckigen Plättchens 94 enden. Das Plättchen 94 begrenzt zusammen mit einem dem Deckteil 73 entsprechenden Deckteil 97 und einem nicht sichtbaren, dem Plättchen 71 entsprechenden Plättchen für jede Elektroden-Gruppe 95 einen Hohlraum zum Aufnehmen einer zu untersuchenden Flüssigkeit. Im übrigen kann der Deckteil 97 mit Einlässen und Auslässen versehen sein, die analog zum Einlass 73b bzw. zum Auslass 73c in einen zugeordneten Hohlraum münden. Der Mehrfach-Sensor 90 ermöglicht zusammen mit einem Messgerät gleichzeitig mehrere Proben zu untersuchen.

Die erfindungsgemässe Messeinrichtung kann noch in verschiedener Hinsicht geändert werden. Zum Beispiel kann man eventuell das aus einem Saphir gebildete Plättchen 54 bzw. 94 durch ein Plättchen aus einem keramischen Material oder einen nicht plättchenförmigen Teil ersetzen.

Bei dem in der Fig. 1 ersichtlichen Sensor kann man den Einlass 73b sowie den Auslass 73c weglassen, so dass der Hohlraum 75 vollständig abgeschlossen ist. Eine zu analysierende Probe kann dann mit einer Pipette oder dergleichen in den durch Entfernen des Deckteils 73 vorübergehend geöffneten Hohlraum eingebracht werden. Entsprechendes gilt auch für den Mehrfach-Sensor 90 gemäss der Fig. 4. Ferner kann man auch einen Mehrfach-Sensor vorsehen, bei dem auf ein und demselben einstückigen, elektrisch isolierenden Plättchen mehr als 4, beispielsweise 6 oder 24 Elektroden-Gruppen vorhanden sind.

Ferner kann man die Deckteile 73, 97 und/oder eventuell die Träger 53, 93 und Elektroden der in den Figuren 1 und 4 ersichtlichen Sensoren durchsichtig ausbilden, so dass die in den Sensoren vorhandenen Zellen auch noch mikroskopisch und/oder mit einer optischen Analysemethode untersucht werden können.

Bei den beschriebenen Ausführungsbeispielen sind die p_{H}-Messelektrode, die Steuerelektrode, die Referenzelektrode und die Gegenelektrode voneinander getrennt angeordnet, so dass keine direkte elektrisch leitende Verbindung zwischen ihnen besteht. Die Messung des p_{H}-Wertes und die Erzeugung Regelung des zum Protonenaustausch dienenden Stromes können dann kontinuierlich und gleichzeitig oder eventuell intermittierend und abwechselnd stattfinden.

Eventuell kann man jedoch die Referenzelektrode für die p_{H}-Messung und die zur Erzeugung eines durch die Flüssigkeit fliessenden Stromes dienende Gegenelektrode durch ein und dieselbe Elektrode bilden. In diesem Fall werden die p_{H}-Messung und die Erzeugung des zum Protonenaustausch dienenden Stromes dann beispielsweise abwechselnd durchgeführt, wobei aber auch eine kontinuierliche, gleichzeitige p_{H}-Messung und Erzeugung sowie Regelung des Stromes möglich sein können.

## Patentansprüche

1. Einrichtung zur Untersuchung des Stoffwechsels von an eine Flüssigkeit angrenzenden Zellen, mit pH-Sensormitteln (56, 58) zum Messen des pH-Wertes der Flüssigkeit, **dadurch gekennzeichnet**, dass eine in Kontakt mit der Flüssigkeit bringbare, zum Protonenaustausch mit der Flüssigkeit ausgebildete Steuerelektrode (57) und eine in Kontakt mit der Flüssigkeit bringbare Gegenelektrode (59) vorhanden sind, dass die pH-Sensormittel (56, 58) , die Steuerelektrode (57) und die Gegenelektrode (59) elektrisch mit elektronischen Schaltungsmitteln verbunden sind und dass die letzteren ausgebildet sind, um einen zwischen der Steuerelektrode (57) sowie der Gegenelektrode (59) durch die Flüssigkeit fliessenden elektrischen Strom zu erzeugen, diesen und/oder eine damit verknüpfte Grösse zu messen und den Strom sowie den Protonenaustausch derart zu regeln, dass der pH-Wert gleich einem Sollwert ist.

2. Einrichtung nach Anspruch 1, dadurch gekennzeichnet, dass die p_{H}-Sensormittel eine in Kontakt mit der Flüssigkeit bringbare p_{H}-Messelektrode (56) aufweisen, deren elektrisches Potential gegenüber der Flüssigkeit vom p_{H}-Wert der letzteren abhängig ist.

3. Einrichtung nach Anspruch 2, dadurch gekennzeichnet, dass ein Träger (53) mit einem elektrisch isolierenden Trägerteil vorhanden ist und dass die p_{H}-Messelektrode (56), die Steuerelektrode (57) und die Gegenelektrode (59) auf dem Trägerteil angeordnete, elektrisch leitende und/oder halbleitende Schichten aufweisen.

4. Einrichtung nach Anspruch 3, dadurch gekennzeichnet, dass die Steuerelektrode (57) die p_{H}-Messelektrode (56) mindestens annähernd umschliesst und dass die Gegenelektrode (59) ausserhalb der Steuerelektrode (57) angeordnet ist.

5. Einrichtung nach Anspruch 4, dadurch gekennzeichnet, dass noch eine in Kontakt mit der Flüssigkeit bringbare, zusammen mit der p_{H}-Messelektrode (56) für die p_{H}-Messung dienende Referenzelektrode (58) vorhanden ist, die ebenfalls am Trägerteil ausserhalb der Steuerelektrode (57) angeordnet ist.

6. Einrichtung nach einem der Ansprüche 3 bis 5, dadurch gekennzeichnet, dass die p_{H}-Messelektrode (56) und/oder die Steuerelektrode (57) eine aus mindestens einem Metalloxid bestehende, mit der Flüssigkeit in Kontakt bringbaren Oxidschicht (66) besitzt.

7. Einrichtung nach Anspruch 6, dadurch gekennzeichnet, dass die Oxidschicht (66) mindestens ein Oxid von mindestens einem der Metalle Iridium, Palladium, Zirkon, Niob, Molybdän, Technetium, Ruthenium, Rhodium, Tantal, Wolfram, Rhenium, Osmium, Platin aufweist und zum Beispiel einkristallin ist.

8. Einrichtung nach einem der Ansprüche 3 bis 7, dadurch gekennzeichnet, dass der Träger (53) zusammen mit mindestens einem mit ihm verbundenen, anderen Teil (71, 73) einen an die genannten Elektroden (56, 57, 58, 59) angrenzenden Raum (75) begrenzt, der vorzugsweise dicht gegen die Umgebung abgeschlossen ist.

9. Einrichtung nach einem der Ansprüche 1 bis 9, dadurch gekennzeichnet, dass die elektronischen Schaltungsmittel ausgebildet sind, um die während einer Messzeit den genannten elektrischen Strom bildende elektrische Ladungsmenge zu ermitteln.

10. Verfahren zur Untersuchung des Stoffwechsels von an eine Flüssigkeit angrenzenden Zellen, insbesondere unter Verwendung einer Einrichtung nach einem der Ansprüche 1 bis 9, wobei der p_{H}-Wert der Flüssigkeit gemessen wird, dadurch gekennzeichnet, dass eine Steuerelektrode (57) und eine Gegenelektrode (59) in Kontakt mit der Flüssigkeit gebracht wird, dass ein Strom zwischen der Steuerelektrode (57) und der Gegenelektrode (59) durch die Flüssigkeit hindurch geleitet und geregelt wird, so dass die Steuerelektrode (59) Protonen mit der Flüssigkeit austauscht, und der p_{H}-Wert gleich einem Sollwert wird, und dass der Strom und/oder eine mit diesem verknüpfte Grösse gemessen wird.

## Claims

1. A device for analyzing the metabolism of cells that border on a liquid, with pH sensing means (56, 58) for measuring the pH value of the liquid, **wherein** are provided a control electrode (57) which can be brought into contact with the liquid and is designed for proton exchange with the liquid, and a counterelectrode (59) which can be brought into contact with the liquid, and wherein the said pH sensing means (56, 58), the said control electrode (57) and the said counterelectrode (59) are electrically connected to electronic circuit elements, and wherein the said circuit elements are designed so as to generate an electric current flowing through the liquid between the said control electrode (57) and the said counterelectrode (59), and to measure this current and/or a variable associated therewith, and to control the current as well as the proton exchange in such a way that the pH value equals a desired preset value.

2. A device as in claim 1, **wherein** the pH sensing means are provided with a pH measuring electrode (56), which can be brought into contact with the liquid and whose electric potential vis-a-vis the liquid is dependent on the pH value of the liquid.

3. A device as in claim 2, **wherein** a supporting base (53) with an electrically insulating part is provided, and wherein the pH measuring electrode (56), the control electrode (57), and the counterelectrode (59) are provided with electrically conductive and/or semi-conductive layers applied on the electrically insulating part.

4. A device as in claim 3, **wherein** the control electrode (57) encircles the pH measuring electrode (56) almost completely, and wherein the counterelectrode (59) is located outside of the control electrode (57).

5. A device as in claim 4, **wherein** a reference electrode (58) is provided, which can be brought into contact with the liquid and which, together with the pH measuring electrode (56), is used for pH measurement, and which is also located on the electrically insulating part outside of the control electrode (57).

6. A device as in any of claims 3 to 5, **wherein** the pH measuring electrode (56) and/or the control electrode (57) are provided with an oxide layer (66) of one or more metal oxides, which can be brought into contact with the liquid.

7. A device as in claim 6, **wherein** the oxide layer (66) consists of at least one oxide of at least one of the metals iridium, palladium, zirconium, niobium, rhodium, tantalum, rhenium, platinum, and is monocrystalline, for example.

8. A device as in any of claims 3 to 7, **wherein** the supporting base (53), together with at least one other part (71, 73) attached thereto, bounds a cavity (75) adjacent to the electrodes (56, 57, 58, 59) referred to above, the said cavity (75) preferably being sealed tight against its environment.

9. A device as in any of claims 1 to 9, **wherein** the electronic circuit elements are designed so as to measure the amount of charge generating the electric current during a measurement period.

10. A method for analyzing the metabolism of cells bordering on a liquid, in particular a method using a device as claimed in any of claims 1 to 9, whereby the pH value of the liquid is determined, **wherein** a control electrode (57) and a counterelectrode (59) are brought into contact with the liquid, and wherein between the said control electrode (57) and the said counterelectrode (59) a current is passed through the liquid and controlled such that the control electrode (57) will exchange protons with the liquid and the pH value will equal a desired preset value, and wherein the current and/or a variable associated therewith is measured.

## Revendications

1. Dispositif d'étude de l'échange de matière de cellules limitrophes d'un liquide avec des moyens de capteur de pH (56, 57) pour mesurer le pH du liquide
caractérisé en ce qu'
il y a une électrode de commande (57) qu'on peut mettre en contact avec le liquide, constituée pour réaliser l'échange protonique avec le liquide et une contre-électrode (59) qu'on peut mettre en contact avec le liquide,
en ce que
les moyens de capteur de pH (56, 58), l'électrode de commande (57) et la contre-électrode (59) sont reliés électriquement aux dispositifs de circuit électronique et que ces derniers sont constitués pour produire un courant électrique s'écoulant dans le liquide entre l'électrode de commande (57) et la contre-électrode (59), pour mesurer ce courant et/ou une grandeur qui en dépend et pour réguler le courant ainsi que l'échange protonique de façon à ce que le pH soit égal à une valeur affichée.

2. Dispositif selon la revendication 1,
caractérisé en ce que
les moyens de capteur de pH présentent une électrode de mesure de pH qu'on peut mettre en contact avec le liquide (56), dont le potentiel électrique vis-à-vis du liquide dépend du pH de ce dernier.

3. Dispositif selon la revendication 2,
caractérisé en ce qu'
un support (53) est présent avec une pièce support isolant électrique, et que l'électrode de mesure de pH (56), l'électrode de commande (57) et la contre-électrode (59) présentent des couches électriquement conductrices et/ou semi-conductrices disposées sur la pièce de support.

4. Dispositif selon la revendication 3,
caractérisé en ce que
l'électrode de commande (57) entoure l'électrode de mesure de pH (56) au moins approximativement et que la contre-électrode (59) est disposée en dehors de l'électrode de commande (57).

5. Dispositif selon la revendication 4,
caractérisé en ce qu'
il existe aussi une électrode des références (58) qui est également sur la pièce support en dehors de l'électrode de commande (57) qu'on peut mettre en contact avec le liquide servant à la mesure du pH avec l'électrode de mesure de pH (56).

6. Dispositif selon l'une des revendications 3 à 5,
caractérisé en ce que
l'électrode de mesure de pH (56) et/ou l'électrode de commande (57) possède une couche d'oxyde (66) constitué d'au moins un oxyde de métal qu'on peut mettre en contact avec le liquide.

7. Dispositif selon la revendication 6,
caractérisé en ce que
la couche d'oxyde (66) comprend au moins un oxyde d'au moins l'un des métaux iridium, palladium, zirconium, niobium, molybdène, technétium, ruthénium, rhodium, tantale, tungstène, rhénium, osmium, platine et est un monocristal par exemple.

8. Dispositif selon l'une des revendications 3 à 7,
caractérisé en ce que
le support (53) avec au moins une autre partie (71, 73) qui lui est liée délimite une chambre limitrophe (75) des électrodes citées (56, 57, 58, 59) qui est close de manière étanche vis-à-vis de l'environnement.

9. Dispositif selon l'une des revendications 1 à 9,
caractérisé en ce que
les moyens de circuits électroniques sont constitués pour mesurer la quantité de charge électrique qui se forme pendant la durée de mesure du courant électrique en question.

10. Procédé d'étude de l'échange de matière de cellules, limitrophes d'un liquide, notamment en utilisant un dispositif selon l'une des revendications 1 à 9, où on mesure le pH du liquide,
caractérisé en ce qu'
on met en contact une électrode de commande (57) et une contre électrode (59) avec le liquide, qu'on fait passer un courant entre l'électrode de commande (57) et la contre électrode (59) dans le liquide, de sorte que l'électrode de commande (59) échange des protons avec le liquide, et que le pH est égal à une valeur affichée et qu'on mesure le courant et/ou une grandeur qui en dépend.
